# FASCICULE DE BREVET EUROPEEN

(11) **EP 0 652 861 B1**
(45) Date de publication et mention de la délivrance du brevet: **03.09.1997**
(21) Numéro de dépôt: 94914529.6
(22) Date de dépôt: 19.05.1994
(51) Int. Cl.: C07C 47/228, C07C 47/232, C07C 43/303, C07C 33/20, C07D 317/12, C07D 313/08, A61K 7/46, C11B 9/00, C07C 45/62, C07C 45/30, C07C 45/51

(54) **COMPOSES AROMATIQUES NOUVEAUX ET LEUR UTILISATION EN PARFUMERIE**
AROMATISCHE VERBINDUNGEN UND IHRE ANWENDUNG IN DER PARFÜMERIE
NOVEL AROMATIC COMPOUNDS AND THEIR USE IN PERFUME PRODUCTS

(30) Priorité: 26.05.1993 CH 1577/93
(43) Date de publication de la demande: 17.05.1995
(73) Titulaire: FIRMENICH SA, 1211 Genève 8 (CH)
(72) Inventeur: WINTER, Beat, 1,chemin Champ-Fleury, CH-1233 Bernex (CH); SKOUROUMOUNIS, George, CH-2000 Neuchatel (CH)
(74) Mandataire: Salvaterra-Garcia, Maria de Lurdes
(86) Numéro de dépôt international: IB9400115
(87) Numéro de publication internationale: WO9427946

(56) Documents cités:
- EP-A- 0 032 659
- EP-A- 0 360 091
- DE-A- 2 720 321
- FR-A- 1 177 132
- GB-A- 2 079 751
- US-A- 3 372 199
- US-A- 3 879 425
- US-A- 4 512 918
- CHEMICAL ABSTRACTS, vol. 071, no. 7, 18 Aoüt 1969, Columbus, Ohio, US; abstract no. 030266, CAGNIANT P ET AL 'Cleavage and migration of tert-butyl groups in Friedel-Crafts reactions. I. Synthesis of tert-butyl-substituted tetralones' cité dans la demande & BULL. SOC. CHIM. FR. (BSCFAS);69; (3); PP.985-91 COLL. SCI. UNIV. METZ;METZ; FR.
- CHEMICAL ABSTRACTS, vol. 071, no. 7, 18 Aoüt 1969, Columbus, Ohio, US; abstract no. 030266, CAGNIANT P ET AL 'Cleavage and migration of tert-butyl groups in Friedel-Crafts reactions. I. Synthesis of tert-butyl-substituted tetralones'
- CHEMICAL ABSTRACTS, vol. 071, no. 7, 18 aout 1969, Columbus, Ohio, US; abstract no. 030266, P. CAGNIANT et al: "Cleavage and migration of tert-butyl groups in Friedel-Crafts reactions. I. Synthesis of tert-butyl-substituted tetralones"

## Description

La présente invention a trait à des acétals et aldéhydes aromatiques nouveaux, utiles à titre d'ingrédients parfumants. Elle concerne, en effet, des composés de formule 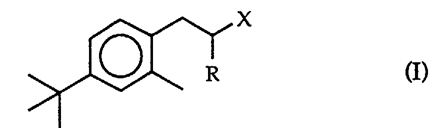 dans laquelle le symbole X représente un groupe - CHO ou un groupe acétal de formule 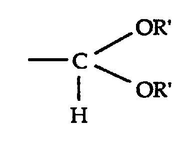 les symboles R', pris isolément, représentant chacun un radical alkyle de C₁ à C₄, linéaire ou ramifié, saturé ou insaturé, ou pris ensemble, représentant un radical alkylène de C₁ à C₄, éventuellement substitué, et le symbole R représente un atome d'hydrogène ou un radical méthyle.

### Technique antérieure

Comme il ressort du tableau suivant, on connaît de l'art antérieur plusieurs composés de structure proche de celle des composés (I), dont certains ont rencontré du succès commercial. Plusieurs de ces composés sont d'ailleurs décrits dans l'oeuvre de référence de S. Arctander, Perfume and Flavor Chemicals, Montclair, N.J., USA (1969) et ceci est indiqué dans le tableau.

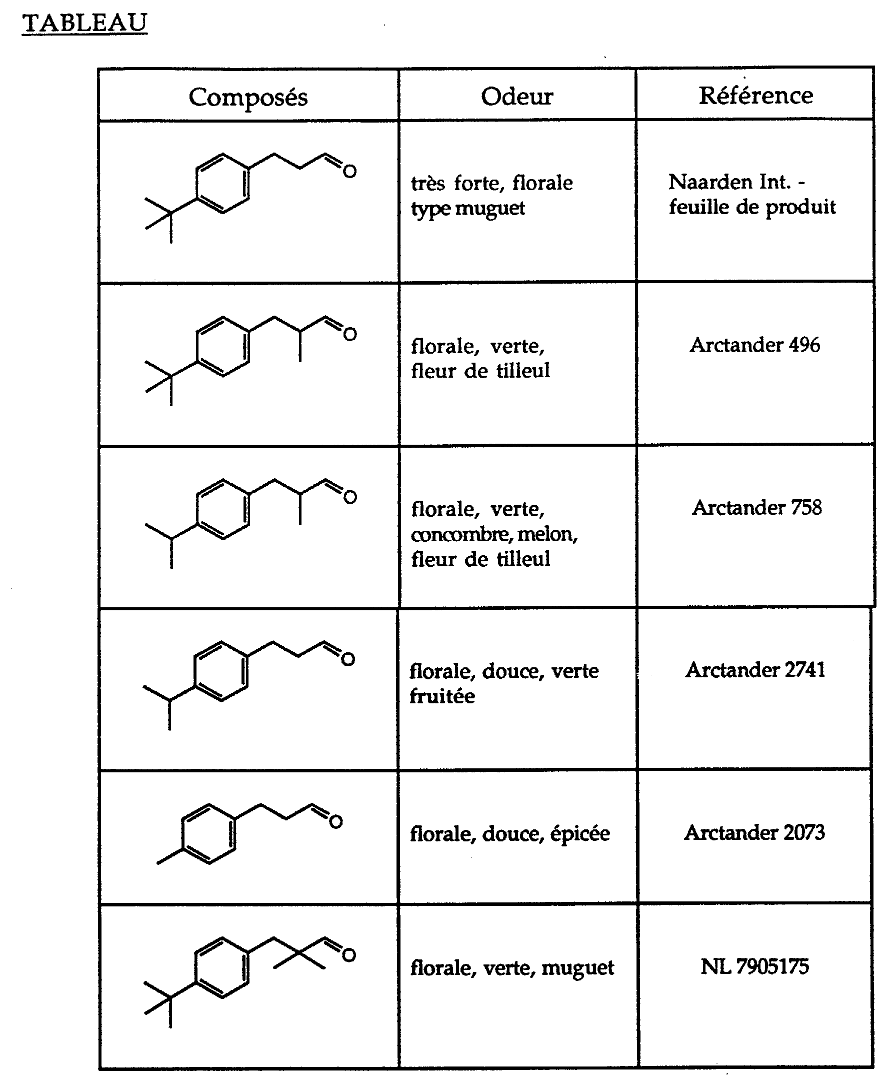

Malgré l'abondance de produits connus de ce type, l'activité de recherche dans ce domaine reste soutenue, notamment dans le but de trouver des composés présentant des nuances odorantes plus variées et aussi des composés dont la stabilité en composition soit améliorée par rapport aux produits connus. En effet, ces aldéhydes connus sont très sensibles à l'oxydation, se transformant facilement en les acides correspondants, lesquels sont soit inodores, soit d'une odeur désagréable, mais en tout cas ne possèdent plus les caractères odorants recherchés.

### Exposé de l'invention

Or nous avons maintenant découvert de façon surprenante que les composés (I) possèdent des propriétés odorantes très utiles et distinctes de celles des produits de l'art antérieur. En particulier, nous avons constaté que le 3-(4-tert-butyl-2-méthyl-1-phényl)propanal selon l'invention permettait de remplacer avec bonheur, dans ses applications typiques, l'aldéhyde p-tert-butyl-α-méthyl-hydrocinnamique ou 3-(4-tert-butyl-1-phényl)-2-méthylpropanal, aussi connu sous le nom de LILIAL® (origine: Givaudan-Roure, Vernier, Suisse), tout en étant, en pratique, d'un emploi plus étendu que celui-ci.

Ce résultat de nos recherches était d'autant plus inattendu que, malgré le grand nombre de composés nouveaux que nous avons préparé et qui possédaient des groupes alkyles ou alkényles dans les différentes positions du squelette benzénique et de la chaîne latérale, seuls les composés (I), et en particulier le dérivé dans lequel R = H, ont montré des caractères odorants exceptionnels. Ces caractères se sont en effet révélés lors de laborieuses évaluations olfactives entreprises par des panels d'experts parfumeurs, lesquels ont découvert avec surprise la supériorité dès qualités odorantes de ces composés, supériorité bien évidente non seulement lors de l'évaluation sur mouillette du produit à l'état pur ou en composition, mais aussi lors de son application pour le parfumage notamment de détergents et adoucissants textiles.

C'est ainsi que nous avons constaté que le 3-(4-tert-butyl-2-méthyl-1-phényl)propanal, le composé préféré de l'invention, développe une odeur réminiscente de celle de l'aldéhyde connu cité plus haut, mais qui possède en plus un caractère anisique, muguet tout à fait distinctif.

Par ailleurs, comme il ressort des exemples présentés plus loin, ce composé selon l'invention présente, vis-à-vis de l'aldéhyde connu cité plus haut, une puissance odorante accrue et une meilleure stabilité contre l'oxydation à l'air. L'invention fournit ainsi un composé nettement plus stable que le LILIAL® connu et qui se prête aussi bien à l'emploi dans des compositions où ce composé connu est typiquement utilisé qu'à la préparation de compositions parfumantes originales.

Les composés de l'invention trouvent un emploi avantageux aussi bien en parfumerie fine, qu'en parfumerie fonctionnelle et de par leurs propriétés odorantes, leur application est bien plus étendue que celle de l'aldéhyde p-tert-butyl-α-méthyl-hydrocinnamique connu. Ils servent à préparer des bases parfumantes et des parfums et sont également fort utiles pour le parfumage d'artides de consommation divers tels les savons, les gels de douche ou bain, les shampoings et produits après-shampoing, les préparations cosmétiques et les désodorisants corporels ou d'air ambiant. D'autre part, grâce à la puissance et à la substantivité de sa note, le 3-(4-tert-butyl-2-méthyl-1-phényl)propanal en particulier s'est révélé d'un emploi très avantageux dans le parfumage de détergents ou adoucissants textiles. Les produits d'entretien peuvent également être parfumés à l'aide des composés (I).

Dans ces applications, on peut les utiliser dans une gamme de concentrations fort étendue. A titre d'exemple on peut citer des concentrations de l'ordre de 5 à 10%, voire même 15 ou 20% en poids, par rapport au poids de la composition dans laquelle ils sont incorporés.

Il est clair cependant que ces valeurs ne peuvent être données qu'à titre indicatif, puisque les concentrations de composé (I) dépendent, et de l'effet olfactif désiré, et de la nature du produit que l'on désire parfumer. D'autre part, elles sont également dépendantes de la nature des autres ingrédients dans une composition donnée, lorsque les composés (I) sont utilisés en mélange avec des solvants, adjuvants et co-ingrédients parfumants d'usage courant. La mention spécifique de tels co-ingrédients est ici superflue. L'état de la technique en présente bien des exemples et l'homme du métier est à même de choisir ceux qui conviendront le mieux à l'effet odorant recherché. On citera comme exemple de référence l'ouvrage de S. Arctander, Perfume and Flavor Chemicals, Montclair, N. J., USA (1969).

Des valeurs bien inférieures à celles citées plus haut, de l'ordre de 0,1 à 0,5% en poids, par rapport au poids de la composition dans laquelle ils sont incorporés, seront normalement utilisées lors de l'emploi des composés (I) dans le parfumage des articles de consommation divers susmentionnés, par exemple savons, détergents et adoucissants textiles.

Les composés (I) sont préparés selon un procédé original qui fait également l'objet de l'invention et qui est caractérisé en ce qu'on fait réagir un alcool de formule 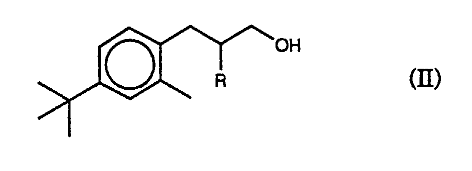 dans laquelle le symbole R représente un atome d'hydrogène ou un radical méthyle, avec un agent oxydant connu pour sa capacité d'oxyder la fonction alcool en aldéhyde, dans un solvant organique inerte, pour obtenir un aldéhyde de formule (I), et, le cas échéant, on transforme cet aldéhyde en l'acétal correspondant, de façon connue en soi.

En tant qu'agent oxydant on peut utiliser un réactif d'usage courant dans les réactions d'oxydation d'alcool en aldéhyde dont on trouve de nombreux exemples dans des ouvrages de référence tels que par exemple le livre de H.O. House, Modern Synthetic Reactions, W.A. Benjamin Inc. 2^{nd} ed., USA (1972).

La réaction aura lieu dans un solvant organique inerte d'usage courant pour ce type de réactions. Des exemples de tels solvants peuvent être trouvés dans l'oeuvre de référence citée.

Les composés de formule (II) sont des alcools aromatiques qui peuvent être préparés à partir de produits commerciaux, selon le schéma que voici : 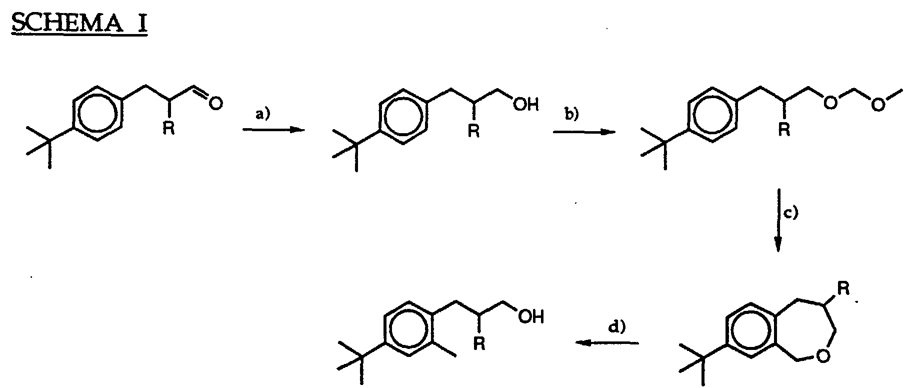 R = H, CH₃
a) LiAlH₄, éther
b) LiBr, CH₂(OCH₃)₂, acide p-toluénesulfonique
c) AlCl₃, CH₂Cl₂
d) 5% Pd-C, acétate d'éthyle, H₂

Une autre synthèse du 3-(4-tert-butyl-2-méthyl-1-phényl)-1-propanol (R = H) a, par ailleurs, été décrite par P. Cagniant et al. dans Bull. Soc. Chim. France, 1969, 985.

Lorsque désiré, les aldéhydes de formule (I) peuvent être transformés en les acétals correspondants à l'aide de méthodes bien connues de l'homme de l'art. De telles méthodes incluent, par exemple, la réaction de l'aldéhyde (I) avec un alcool ou un diol approprié, en présence d'un catalyseur acide [voir, par exemple, J. March, Advanced Organic Chemistry, Reactions, Mechanisms & Structure, section 6-6, 3ème éd., John Wiley & Sons, USA (1985)].

Alternativement, les composés de formule (I) peuvent être préparés selon un autre procédé original, caractérisé en ce qu'on soumet à une hydrogénation catalytique, dans un solvant organique inerte, un aldéhyde de formule 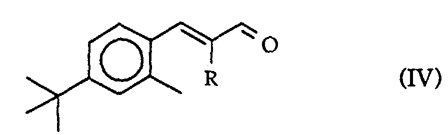 dans laquelle R a le sens indiqué à la formule (I) et, le cas échéant, on acétalyse, de façon connue en soi, l'aldéhyde (I) ainsi obtenu.

La réaction d'hydrogénation a lieu en présence d'un catalyseur tel que le Pd-C, dans les conditions décrites plus loin.

Les produits de départ de formule (IV) sont des composés nouveaux, préparés à partir de l'o-xylène, selon le schéma que voici:

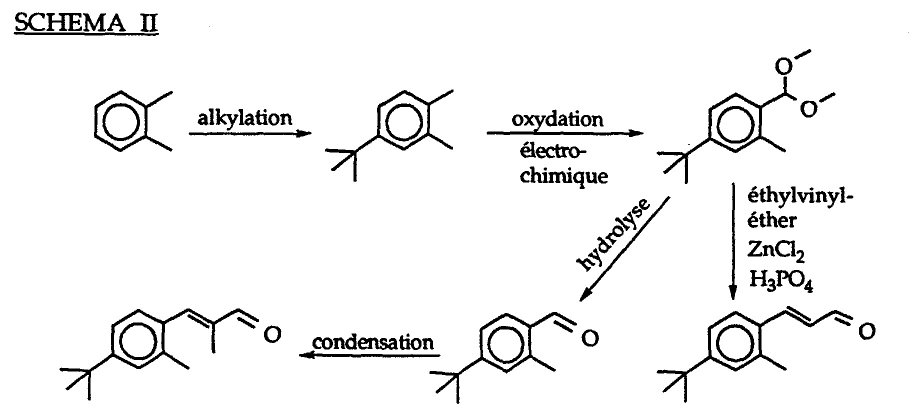

La première étape de ce procédé est une alkylation classique, effectuée en présence de quantités catalytiques de AlCl₃ [voir P. Cagniant et al., réf. citée]. Le 4-tert-butyl-1,2-diméthylbenzène ainsi obtenu est ensuite oxydé électrochimiquement en l'acétal correspondant. Ce dernier a ensuite été transformé en l'aldéhyde (IV) non méthylé dans la chaîne à l'aide d'une réaction de condensation de type Müller-Cunradi [voir, par exemple, U. von der Bruggen et al., J. Org. Chem. 53, 2920 (1988) et références y citées], à l'aide d'un acide de Lewis, par exemple le ZnCl₂, mais effectuée en présence d'acide phosphorique.

Les conditions dans lesquelles les réactions illustrées aux schémas I et II ont été effectuées sont décrites plus en détail dans les exemples de préparation présentés ci-après, dans lesquels les températures sont indiquées en degrés centigrades et les abréviations ont le sens usuel dans l'art.

L'invention sera également illustrée de façon plus détaillée à l'aide des exemples d'application en parfumerie présentés plus loin.

### Description sommaire du dessin

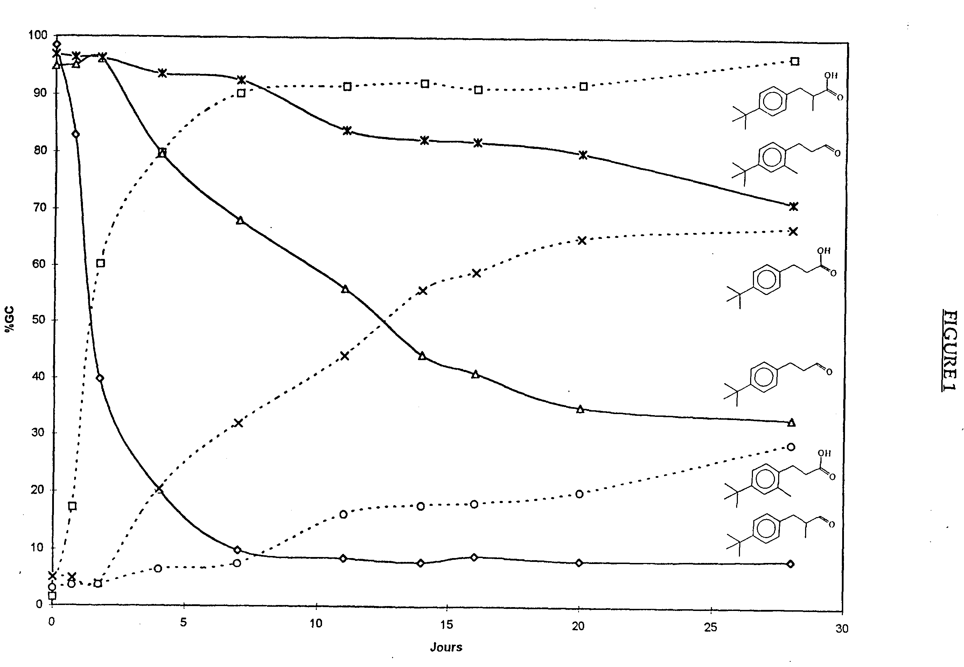

La Figure 1 représente le graphique ayant trait à l'expérience décrite à l'Exemple 18.

### Manières de réaliser l'invention

### Exemple 1

### Préparation de 3-(4-tert-butyl-2-méthyl-1-phényl)propanal

### a) Méthode selon le schéma I

A une suspension de chlorochromate de pyridinium (PCC, 13,4 g, 62,0 mmole) dans le CH₂Cl₂ (80 ml) on a ajouté à température ambiante une solution de 3-(4-tert-butyl-2-méthyl-1-phényl)-1-propanol (9,1 g, 44 mmole) dans CH₂Cl₂ (20 ml). Après 15 h, le mélange a été dilué à l'éther (200 ml), filtré et passé à travers une colonne de Florisil® (adsorbant pour la chromatographie ; origine : Fluka, Suisse). L'éluant a été concentré et distillé pour fournir le propanal désiré avec une pureté de 90% (rend. 54%).
P.f. 81-83°, recristallisé (-30°/éther de pétrole)
- IR(pur) :: 2960, 2900, 2860, 2810, 2720, 1725, 1610, 1510, 1460, 1390, 1270, 1140, 1110, 1040, 885, 830 cm⁻¹
- RMN(¹H, 360MHz, CDCl₃) :: 9,83(large, 1H) ; 7,18(s, 1H) ; 7,16(d, J=9Hz, 1H) ; 7,06(d, J=9Hz, 1H) ; 2,95-2,87(m, 2H) ; 2,76-2,68(m, 2H) ; 2,31(s, 3H) ; 1,30(s, 9H) δ ppm
- RMN(¹³C, 90,5MHz, CDCl₃) :: 201,8(d) ; 149,3(s) ; 135,4(s) ; 135,4(s) ; 128,2(d) ; 127,4(d) ; 123,1(d) ; 44,0(t) ; 34,3(s) ; 31,4(3q) ; 25,0(t) ; 19,6(q) δ ppm
- SM :: 204(18), 189(100), 171(10), 161(9), 145(75), 133(15), 131(19), 130(20), 119(15), 115(18), 105(23), 91(18), 87(3), 77(8), 65(6), 57(14), 41(13)
Odeur décrite plus haut.

Le 3-(4-tert-butyl-2-méthyl-1-phényl)-1-propanol de départ a été préparé selon le schéma I, à partir de 3-(4-tert-butyl-1-phényl)propanal (origine : Quest Int.). On a procédé comme suit.
Sous N₂ et à température ambiante, 0,5 moles de 3-(4-tert-butyl-1-phényl)propanal dans le diéthyléther ont été ajoutés lentement à une suspension de LiAlH₄ (0,5 éq. mol) dans l'éther. La réaction a été suivie par chromatographie en couche mince et était complète 15 minutes après la terminaison de l'introduction. Le mélange réactionnel a été refroidi à 0° et traité avec une solution 1 N en NaOH (volume de solution = 5 fois le poids de LiAlH₄ utilisé dans la réaction). La phase éthérée a été filtrée et l'évaporation du solvant a fourni le 3-(4-tert-butyl-1-phényl)-1-propanol (rend. 83%, pureté 88%).
- IR(pur) :: 3320, 2940, 2850, 1500, 1450, 1405, 1385, 1365, 1260, 1050, 1010, 825 cm⁻¹
- RMN(¹H, 360MHz, CDCl₃) :: 7,31(d, J=9Hz, 2H) ; 7,14(d, J=9Hz, 2H) ; 3,72-3,63(m, 2H) ; 2,71-2,64(m, 2H) ; 1,94-184(m, 2H) ; 1,56-1,50(large, 1H) ; 1,32(s, 9H) δ ppm
- RMN(¹³C, 90,5MHz, CDCl₃) :: 148,7(s) ; 138,8(s) ; 128,1(2d) ; 125,3(2d) ; 62,3(t) ; 34,3(s) ; 34,2(t) ; 31,5(t) ; 31,4(3q) δ ppm
- SM :: 192(15), 177(100), 159(13), 147(4), 131(53), 117(24), 105(12), 91(28), 77(6), 57(12), 41(14)

Le propanol susmentionné a ensuite été transformé en le 1-tert-butyl-4-(3-méthoxyméthoxypropyl)benzène à l'aide de diméthoxyméthane, de façon analogue à celle décrite par J.L. Gras et al. dans Synthesis 74, (1985) mais en utilisant 12 équivalents de diméthoxyméthane pour 1 équivalent d'alcool. Le méthoxyméthyléther susmentionné a été ensuite transformé comme il est décrit par A. Rieche et al., Chem. Ber. 95, 91 (1962) mais en utilisant comme solvant le dichlorométhane. La concentration de méthoxyméthyléther dans ce solvant a varié entre 0,1 et 0,3 M. Le produit de la réaction a été purifié par chromatographie pour fournir la 8-tert-butyl-1,3,4,5-tétrahydro-2-benzoxépine (rend. 67%, pureté >99%).
P.éb.92°/16 Pa
P.f.49°
- IR(pur) :: 2940, 2880, 2820, 1500, 1445, 1430, 1355, 1250, 1220, 1100, 1095, 1030, 995, 970, 910, 895, 880, 830, 815, 750, 730, 670 cm⁻¹
- RMN(¹H, 360MHz, CDCl₃) :: 7,22-7,13(m, 2H) ; 7,10(d, J=7,2Hz, 1H) ; 4,66(s, 2H) ; 4,07-4,02(m, 2H) ; 2,99-2,93(m, 2H) ; 1,87-1,80(m, 2H) ; 1,30(s, 9H) δ ppm
- RMN(¹³C, 90,5MHz, CDCl₃) :: 149,0(s) ; 139,6(2s) ; 128,9(d) ; 125,7(d) ; 124,5(d) ; 75,7(t) ; 75,6(t) ; 35,0(t) ; 34,3(s) ; 31,4(3q) ; 30,5(t) δ ppm
- SM:: 204(17), 189(100), 171(15), 147(24), 145(55), 131(21), 115(22), 105(24), 91(28), 77(12), 71(9), 65(10), 57(25), 51(4), 41(20)
- Odeur:: florale, fleurs blanches, poussière, chimique.

La tétrahydrobenzoxépine susmentionnée a été dissoute dans l'acétate d'éthyle (solution 1,5 M) et agitée à température ambiante avec du Pd/C à 5% (1% poids/poids par rapport à la benzoxépine) sous hydrogène. Une fois la réaction complétée (quelques heures), le catalyseur a été filtré et le solvant évaporé à pression réduite pour fournir le 3-(4-tert-butyl-2-méthyl-1-phényl)-1-propanol désiré pur à 88% (rend. 83%).
P. éb.110°/13 Pa
- IR(pur) :: 3300, 2940, 2850, 1500, 1460, 1350, 1270, 1050, 1030, 820, 810 cm⁻¹
- RMN(¹H, 360MHz, CDCl₃) :: 7,18-7,11(m, 2H) ; 7,07(d, J=9Hz, 1H) ; 3,7(t, J=7,2Hz, 2H) ; 2,68-2,64(m, 2H) ; 2,32(s, 3H) ; 1,89-1,79(m, 2H) ; 1,6(large, 1H) ; 1,31(s, 9H) δ ppm
- RMN(¹³C, 90,5MHz, CDCl₃) :: 148,8(s) ; 138,9(s) ; 135,4(s) ; 128,5(d) ; 127,2(d) ; 122,8(d) ; 62,6(t) ; 34,2(s) ; 33,0(t) ; 31,4(3q) ; 29,0(t) ; 19,6(t) δ ppm
- SM :: 206(23), 191(65), 173(15), 161(5), 145(100), 131(90), 119(18), 117(17), 115(20), 106(24), 105(47), 91(30), 77(15), 72(4), 65(8), 57(42), 44(12), 41(35)
- Odeur :: florale.

### Méthode selon la schéma II

On a ajouté à 360 g (1,78 mole) de 3-(4-tert-butyl-2-méthyl-1-phényl)-2-propénal dans l'éthanol (1 kg) du Pd-C à 5% (3 g) et de l'acétate de potassium (360 g, 2 mmole). Le mélange a été hydrogéné à 40° et à une pression de 4x10⁵ Pa pendant 24h. Après filtration du catalyseur et évaporation du solvant, on a distillé sous vide (colonne Vigreux de 22 cm) pour obtenir le 3-(4-tert-butyl-2-méthyl-1-phényl)propanal désiré (310 g, pur à 93%, rend. 85%). L'analyse de ce composé a fourni les mêmes résultats que ceux cités sous a).

L'aldéhyde insaturé de départ a été préparé selon le schéma II, comme suit.
On a ajouté à de l'o-xylène (Fluka purum, 905 ml, 7,5 mole), maintenu sous agitation, à 0°, du AlCl₃ (6,7 g, 50 mmole), et ensuite, goutte à goutte, du chlorure de tert-butyle (Fluka puriss., 551 ml, 5 mole), pendant 1,25 h, en maintenant la température entre 0 et 5° (le HCl libéré a été piégé dans NaOH 2,5N). On a laissé la température monter jusqu'à température ambiante. Après 60 h, on a versé sur un mélange de glace et éther, lavé la phase organique successivement avec de la saumure (2x), H₂O, NaHCO₃ sat. et saumure, séché sur Na₂ SO₄ et distillé sous vide (colonne Vigreux, 30 cm) pour obtenir du 4-tert-butyl-1,2-diméthylbenzène (pureté > 99%, rend. 90%).
P.éb.92-95°/17x10² Pa
- IR(pur) :: 3030, 2980, 2890, 1520, 1470, 1455, 1370, 1280, 1150, 825 cm⁻¹
- RMN(¹H, 360MHz, CDCl₃) :: 7,16(s large, 1H) ; 7,13(d large, J=8, 1H) ; 7,06(d, J=8, 1H) ; 2,27(s, 3H) ; 2,23(s, 3H) ; 1,30(s,9H) δ ppm
- RMN(¹³C, 90,5MHz, CDCl₃) :: 148,7(s) ; 136,0(s) ; 133,5(s) ; 129,4(d) ; 126,7(d) ; 122,7(d) ; 34,2(s) ; 31,5(3q) ; 20,0(q) ; 19,1(q) δ ppm
- SM :: 162(M⁺,28), 147(100), 131(6), 119(43), 107(22), 91(17), 77(7), 65(4), 41(6)

Le 4-tert-butyl-1,2-diméthylbenzène (430 g, 265 moles) a été oxydé électrochimiquement à une température d'environ 35°, dans un réacteur inox, en solution dans le méthanol (21, 1580 g), en utilisant du p-toluènesulfonate de sodium comme électrolyte et une cellule de type EBERSON/WITMER, à électrodes de carbone, en faisant passer un courant de 29 mA/cm², pendant 18,5 h. On a obtenu 564,5 g de 4-tert-butyl-1-(diméthoxyméthyl)-2-méthylbenzène, pur à 75%. Ce produit brut a été purifié par distillation (p. éb. 119-126°/13 hPa) pour fournir un liquide incolore pur à 77% (rend. 66%) qui a été utilisé tel quel dans l'étape suivante.
- IR(pur) :: 2950, 2890, 1600, 1445, 1350, 1220, 1185, 1110, 1090, 1050, 970, 820 cm⁻¹
- RMN(¹H, 360MHz, CDCl₃) :: 7,44 (d,J=8, 1H) ; 7,21(dd, J₁=8, J₂=2, 1H) 7,16(d, J=2, 1H) ; 5,42(s,1H) ; 3,32(s, 6H) ; 2,36(s,3H) ; 1,30(s,9H) δ ppm
- RMN(¹³C, 90,5MHz, CDCl₃) :: 151,2(s) ; 135,6(s) ; 132,8(s) ; 127,6(d) ; 126,3(d) ; 122,3(d) ; 102,1(d) ; 53,1(2q) ; 34,4(s) ; 31,3(3q) ; 19,1(q) δ ppm
- SM :: 222(M⁺,2), 191(100), 176(16), 161(14), 133(10), 115(7), 105(13), 91(9), 75(8), 65(3), 41(5)
- Odeur :: verte, cire, légèrement cuminique, pétrole

On a ajouté à l'acétal susmentionné (708 g, 2,6 mole), à -10°, rapidement et sous agitation, une solution de ZnCl₂ (14,2 g, 104 mmole), dans l'acétate d'éthyle (136 ml). Après 5 min, on a ajouté du H₃PO₄ à 85% (1,23 ml, 182 mmole) et refroidi la solution, devenue jaune, à -20°. Après 15 min, on a ajouté goutte à goutte, de l'éthylvinyléther (Fluka purum, 377 ml, 3,9 mole) pendant 1,5 h, en maintenant la température entre 0 et 5°. A la fin de l'addition, la couleur du mélange réactionnel était devenue violette. Après 1 h à 0° et 15 h à température ambiante, l'analyse chromatographique indiquait la formation de 73% des acétals intermédiaires. Ce mélange brut d'acétals a été ajouté au moyen d'une canule à un mélange d'acide formique (650 ml), formiate de sodium (213 g) et eau (338 ml), et le tout chauffé pendant 3 h avec un bain à 110°, en distillant continuellement les volatiles (p. éb. 90°/10⁶ Pa). Après 1 h à 110° et 15 h à température ambiante, le mélange solidifié a été dilué à l'eau (250 ml) et éther de pétrole 30-50° pour extraction. On a lavé la phase organique, séché et concentré. Le produit brut (667 g, pur à 68%, rend. 86%) a été cristallisé plusieurs fois dans l'éther de pétrole 30-50°, à 0°, pour fournir le 3-(4-tert-butyl-2-méthyl-1-phényl)-2-propénal avec une pureté supérieure à 99% (rend. 78%).
P.f. 75-77°
- IR(CHCl₃) :: 2950, 1665, 1595, 1140, 1095, 965 cm⁻¹
- RMN(¹H, 360MHz, CDCl₃) :: 9,70(d, J=8, 1H) ; 7,75 (d, J=16, 1H) ; 7,54(d, J=8, 1H) ; 7,28(d large, J=8, 1H) ; 7,25(s large, 1H) ; 6,65(dd, J₁=16, J₂=8, 1H) ; 2,48(s, 3H) ; 1,32(s, 9H) δ ppm
- RMN(¹³C, 90,5MHz, CDCl₃) :: 193,9(d) ; 154,7(s) ; 150,2(d) ; 137,7(s) ; 130,1(s) ; 128,8(d) ; 128,1(d) ; 126,8(d) ; 123,7(d) ; 34,8(S) ; 31,1(3q) ; 20,0(q) δ ppm
- SM :: 202(M⁺,10), 87(100), 159(9), 145(92), 128(15), 115(22), 105(4), 91(10), 77(5), 55(8), 41(8)
- Odeur :: aldéhydique, métallique

### Exemple 2

### Préparation de 3-(4-tert-butyl-2-méthvl-1-phényl)-2-méthylpropanal

On a procédé de façon identique à celle décrite dans l'exemple 1 a) mais en utilisant comme produit de départ le 3-(4-tert-butyl-2-méthyl-1-phényl)-2-méthyl-1-propanol. Le 3-(4-tert-butyl-2-méthyl-1-phényl)-2-méthylpropanal a été obtenu avec une pureté supérieure à 99% (rend. 68%).
P.éb. 100-l10°/9 Pa
- IR(pur) :: 3400, 2940, 2820, 2800, 2690, 1710, 1600, 1450 cm⁻¹
- RMN(¹H, 360MHz, CDCl₃) :: 9,71(d, J=2,9Hz, 1H) ; 7,17(s, 1H) ; 7,16(d, J=7,9Hz) ; 7,04(d, J=7,9Hz, 1H) ; 3,06(dd, J₁=14,4Hz, J₂=7,2Hz, 1H) ; 2,71-2,59(m, 1H) ; 2,53(dd, J₁=14,4Hz, J₂=9Hz, 1H) ; 2,31(s, 3H) ; 1,30(s, 9H) ; 1,11(d, J=6,7Hz) δ ppm
- RMN(¹³C, 90,5MHz, CDCl₃) :: 149,3(s) ; 204,2(d) ; 135,5(s) ; 134,0(s) ; 129,4(d) ; 127,4(d) ; 122,9(d) ; 46,8(d) ; 34,2(s) ; 33,5(t) ; 31,4(3q) ; 19,7(q) ; 13,5(q) δ ppm
- SM :: 218(18), 203(57), 185(8), 173(3), 161(100), 145(27), 133(23), 131(24), 119(13), 105(16), 91(16), 77(8), 57(15), 41(13)
- Odeur :: florale, verte, aromatique, fenchylique.

Le 3-(4-tert-butyl-2-méthyl-1-phényl)-2-méthyl-1-propanol de départ a été préparé de façon identique à celle décrite dans l'exemple 1 a) pour son homologue non méthylé dans la chaîne, mais en partant du 3-(4-tert-butyl-1-phényl)-2-méthylpropanal (origine : Givaudan-Roure). Les données analytiques des produits intermédiaires étaient les suivantes:
3-(4-tert-butyl-1-phényl)-2-méthyl-1-propanol (pureté : 99%)
Rend. 99%
- IR(pur) :: 3340, 2960, 2870, 1460, 1360, 1270, 1040, 850 cm⁻¹
- RMN(¹H, 360MHz, CDCl₃) :: 7,30(d, J=8Hz, 2H) ; 7,10(d, J=8Hz, 2H) ; 3,53(dd, J₁=11Hz, J₂=6Hz, 1H) ; 3,46(dd, J₁=11Hz, J₂=6Hz, 1H) ; 2,71(dd, J₁=14Hz, J₂=6Hz, 1H) ; 2,40(dd, J₁=14Hz, J₂=8Hz, 1H) ; 1,93(m, 1H) ; 1,44(s, 1H) ; 1,3l(s, 9H) ; 0,92(d, J=7Hz, 3H) δ ppm
- RMN(¹³C, 90,5MHz, CDCl₃) :: 148,8(s) ; 137,6(s) ; 128,8(2d) ; 125,2(2d) ; 67,8(t) ; 39,3(t) ; 37,8(t) ; 34,4(s) ; 31,4(3q) ; 16,6(q) δ ppm
- SM :: 206(16), 191(100), 173(11), 159(3), 147(30), 131(28), 117(25), 105(15), 91(30), 77(8), 65(5), 57(22), 41(14)

1-tert-butyl-4-(2-méthyl-3-méthoxyméthoxypropyl)benzène (pureté : >99%)
Rend. 87%
- IR(pur) :: 2940, 2910, 1450, 1355, 1260, 1145, 1105, 1040, 915 cm⁻¹
- RMN(¹H, 360MHz, CDCl₃) :: 7,29(d, J=7,9Hz, 2H) ; 7,09(d, J=7,0Hz, 2H) ; 4,63(s, 2H); 3,44-3,33(m, 2H) ; 3,37(m, 3H) ; 2,76(dd, J₁=14,4Hz, J₂=7,2Hz, 1H) ; 2,37(dd, J₁=14,4Hz, J₂=7,2Hz, 1H) ; 2,09-1,96(m, 1H) ; 1,31(s, 9H) ; 0,92(d, J=6,7Hz, 3H) δ ppm
- RMN(¹³C, 90,5MHz, CDCl₃) :: 148,6(s) ; 137,6(s) ; 128,9(d) ; 128,8(d) ; 125,1(2d) ; 96,7(t) ; 72,8(t) ; 55,2(q) ; 39,5(t) ; 35,5(d) ; 34,4(s) ; 31,5(3q) ; 17,0(q) δ ppm
- SM :: 250(3), 218(19), 203(65), 188(5), 173(34), 147(100), 145(23), 132(25), 131(84), 117(32), 105(17), 91(30), 77(6), 57(73), 45(58)

8-tert-butyl-1,3,4,5-tétrahydro-4-méthyl-2-benzoxépine (pureté : >99%)
Rend. 76%
P.f.50-51°C
- IR(pur) :: 3030, 2940, 2860, 1495, 1350, 1113, 1090 cm⁻¹
- RMN(¹H, 360MHz, CDCl₃) :: 7,20(dd, J₁=7,94Hz, J₂=1,8Hz, 1H) ; 7,16(d, J=1,8Hz, 1H) ; 7,09(d, J=7,94Hz, 1H) ; 4,69(s, 2H) ; 4,07(dd, J₁=10,8Hz, J₂=6Hz, 1H) ; 3,59(dd, J₁=10,8Hz, J₂=3,6Hz) ; 2,87-2,81 (m, 2H) ; 2,0-1,9(m, 1H) ; 1,3l(s, 9H) ; 0,89(d, J=6,71Hz, 3H) δ ppm
- RMN(¹³C, 90,5MHz, CDCl₃) :: 149,1(s) ; 139,4(s) ; 137,8(s) ; 129,5(d) ; 125,5(d) ; 124,5(d) ; 81,5(t) ; 75,5(t) ; 42,7(t) ; 34,3(s) ; 34,2(s) ; 31,4(3q) ; 17,8(q) δ ppm
- SM :: 218(29), 203(100), 185(12), 173(24), 161(54), 145(41), 133(22), 131(25), 115(23), 105(21), 91(32), 77(12), 65(10), 57(32), 41(20)

3-(4-tert-butyl-2-méthyl-1-phényl)-2-méthyl-1-propanol (pureté : 99%)
Rend. 99%
P.éb. 130-140°C/3-7 Pa
- IR(pur) :: 3600, 3500, 2920, 1590, 1440, 1020 cm⁻¹
- RMN(¹H, 360MHz, CDCl₃) :: 7,15(s, 1H) ; 7,14(d, J=9Hz, 2H) ; 7,04(d, J=9Hz, 2H) ; 3,57(dd, J₁=11Hz, J₂=6,1Hz, 1H) ; 2,71(dd, J₁=14,0Hz, J₂=6,7Hz, 1H) ; 2,38(dd, J₁=14,0Hz, J₂=9Hz, 1H) ; 2,31(s, 3H) ; 1,98-1,87(m, 1H) ; 1,45(s, 9H) ; 0,95(d, J=6,7Hz, 3H) δ ppm
- RMN(¹³C, 90,5MHz, CDCl₃) :: 148,9(s) ; 135,9(s) ; 135,6(s) ; 129,6(d) ; 127,3(d) ; 122,6(d) ; 68,1(t) ; 36,7(s) ; 36,7(t) ; 34,2(s) ; 31,4(3q) ; 19,8(q) ; 16,8(q) δ ppm
- SM :: 220(25), 205(91), 187(8), 173(4), 161(100), 145(33), 131(35), 119(22), 115(15), 105(28), 77(9), 57(25), 41(17)

### Exemple 3

### Préparation de 4-tert-butyl-1-(3,3-diméthoxypropyl)-2-méthylbenzène

A une solution de 3-(4-tert-butyl-2-méthyl-1-phényl)propanal (1,06 g, 5 mmole) dans le méthanol (10 ml), à température ambiante, on a ajouté HCl conc. (3 gouttes). Après 3 h, on a versé la solution dans un mélange d'éther et NaHCO₃ sat. pour extraction. On a lavé la phase organique avec NaHCO₃, séché sur K₂CO₃ et concentré. Après distillation au four à boules, on a obtenu le produit désiré, dont la pureté était de 89% lequel présentait les caractères analytiques suivants:
- IR(pur) :: 2960, 2870, 1605, 1500, 1460, 1385, 1360, 1270, 1195, 1130, 1085, 1060, 990, 960, 920, 885, 830 cm⁻¹
- RMN(¹H, 360MHz, CDCl₃) :: 7,21(m, 2H) ; 7,13 (d, J=8, 1H) ; 4,48(t, J=6, 1H) ; 3,40(s, 6H) ; 2,68(m, 2H) ; 2,37(s, 3H) ; 1,93(m, 2H) ; 1,35(s, 9H) δ ppm
- RMN(¹³C, 90,5MHz, CDCl₃) :: 148,8(s) ; 136,7(s) ; 135,4(s) ; 128,4(d) ; 127,2(d) ; 122,8(d) ; 104,0(d) ; 52,6(2q) ; 34,2(s) ; 32,8(t) ; 31,4(3q) ; 27,7(t) ; 19,6(q) δ ppm
- SM :: 250(M⁺,2), 235(2), 218(20), 203(41), 171(53), 161(78), 131(100), 102(24), 75(87), 57(16), 41(9)
- Odeur :: florale, muguet, aldéhydée

### Exemple 4

### Préparation de 2-[2-(4-tert-butyl-2-méthyl-1-phényl)éthyl]-1,3-dioxolane

Un mélange de 3-(4-tert-butyl-2-méthyl-1-phényl)propanal (2,11 g, 9,8 mmole), d'éthylèneglycol (6,1 g, 98mmole) et d'acide p-toluènesulfonique (95 g, 0,5 mmole) dans le cyclohexane (25 ml), a été chauffé à reflux (80°) pendant 3 h avec une trappe de type Dean-Stark. Le mélange refroidi a été versé sur éther et NaHCO₃ aq. sat., et la phase organique lavée avec NaHCO₂ aq. sat., séchée sur K₂CO₃ et concentrée. Après distillation au four à boules, on a obtenu le dioxolane désiré dont la pureté était de 94% présentant les caractères analytiques suivants:
- IR(pur) :: 2950, 2860, 1600, 1500, 1450, 1400, 1385, 1350, 1140, 1125, 1050, 1030 cm⁻¹
- RMN(¹H, 360MHz, CDCl₃) :: 7,15(m, 2H) ; 7,09(d, J=8, 1H) ; 4,92(t, J=5, 1H) ; 4,00(m, 2H) ; 3,88(m, 2H) ; 2,70(m, 2H) ; 2,32(s, 3H) ; 1,94(m, 2H) ; 1,31(s, 9H) δ ppm
- RMN(¹³C, 90,5MHz, CDCl₃) :: 148,8(s) ; 136,7(s) ; 135,4(s) ; 128,3(d) ; 127,2(d) ; 122,8(d) ; 104,1(d) ; 64,9(2t) ; 34,2(s+t) ; 31,4(3t) ; 27,0(t) ; 19,5(q) δ ppm
- SM :: 248(M⁺,9), 223(10), 186(19), 171(28), 161(9), 145(20), 131(24), 115(15), 106(39), 100(100), 91(15), 87(19), 73(92), 57(30), 45(34), 41(13), 29(17)
- Odeur :: florale

### Exemple 5

### Préparation d'une composition parfumante

On a préparé une composition parfumante de base destinée à un parfum de type féminin en mélangeant les ingrédients suivants :

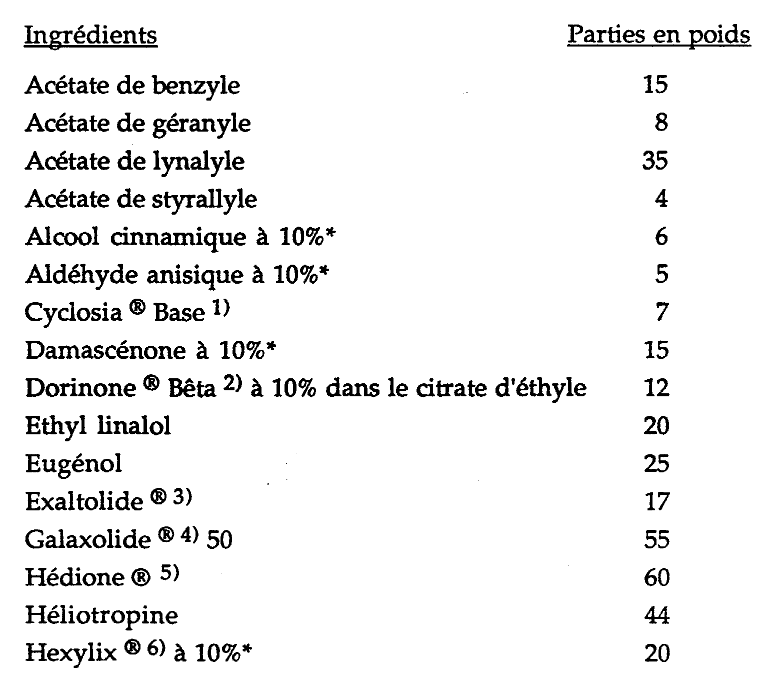

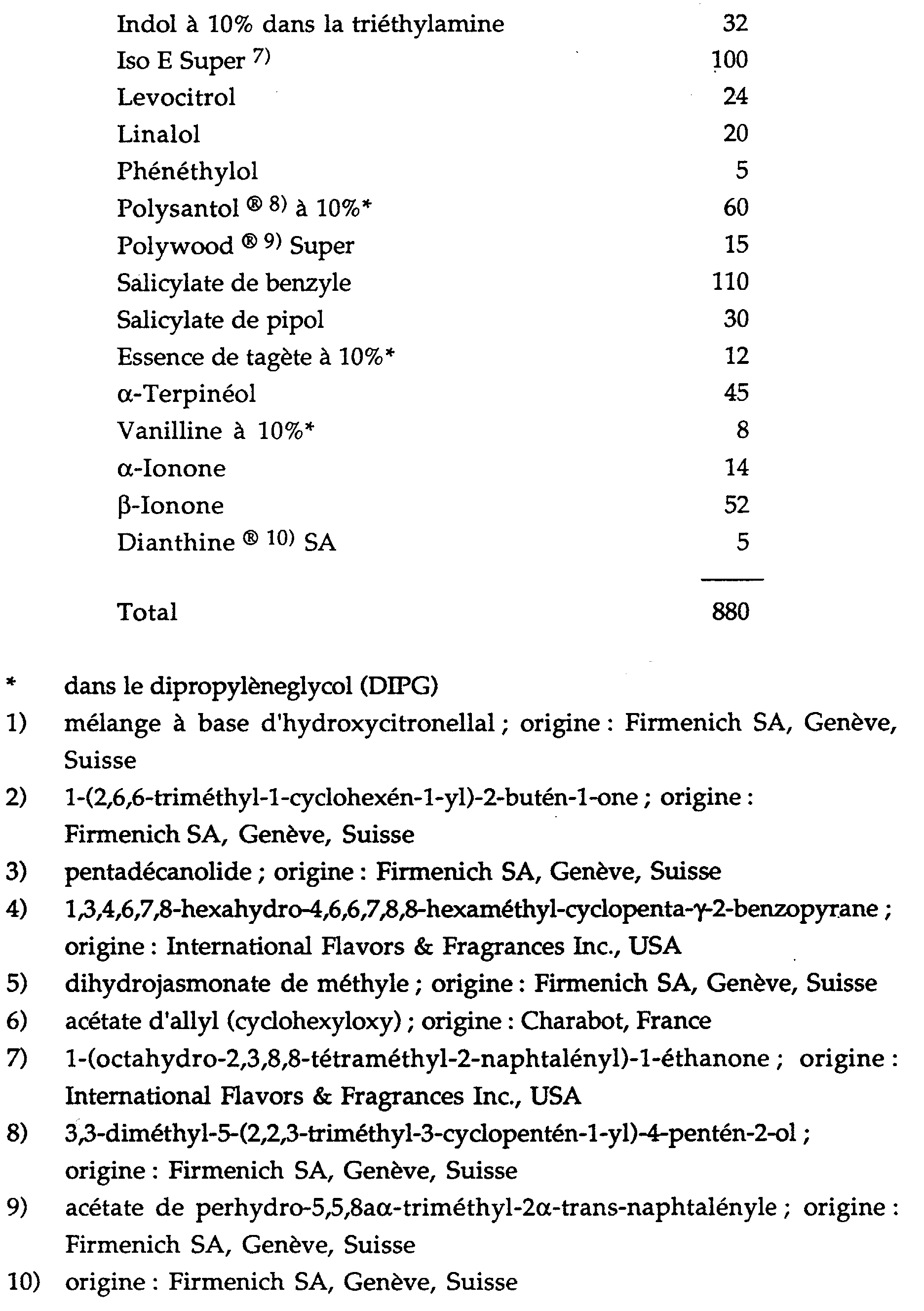

A cette composition de base de type florale, verte, on a ajouté d'une part 120 parties en poids de 3-(4-tert-butyl-2-méthyl-1-phényl)propanal selon l'invention pour préparer une composition nouvelle A et, d'autre part, 120 parties en poids de 3-(4-tert-butyl-1-phényl)-2-méthylpropanal pour préparer une composition B.
Les deux compositions ont ensuite été évaluées à l'aveugle par un panel de 13 experts parfumeurs. De l'avis unanime de ces derniers, la composition nouvelle A a été préférée pour sa note florale beaucoup plus douce et naturelle que celle de la composition B. Les parfumeurs ont aussi trouvé que l'odeur de la composition A était plus puissante et avait plus de volume, la note odorante apparaissant bien plus poudrée et les caractères jasminé et muguet étant nettement exaltés.

### Exemple 6

### Préparation d'une composition parfumante

On a préparé une composition parfumante de base, destinée à un détergent en poudre, par mélange des ingrédients suivants:

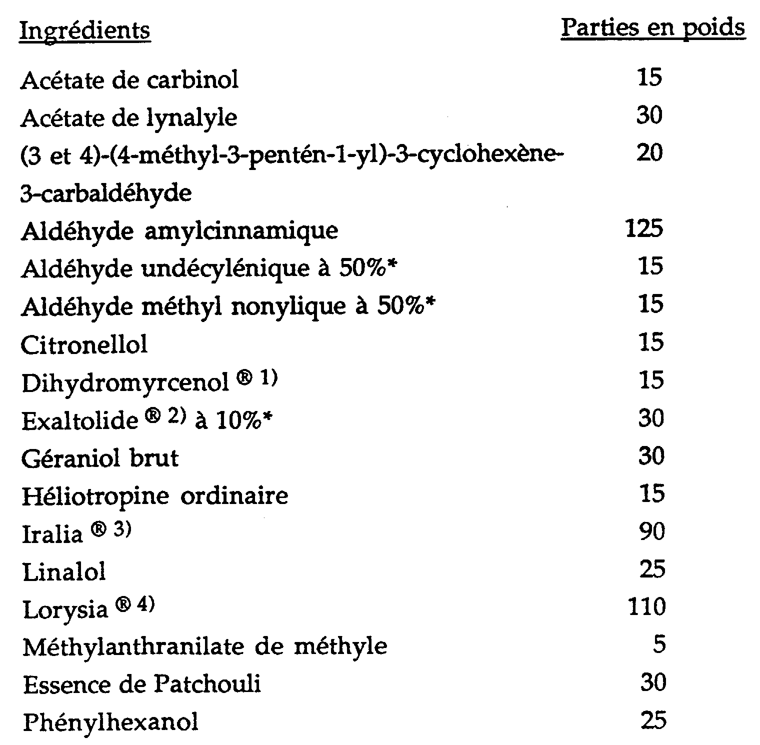

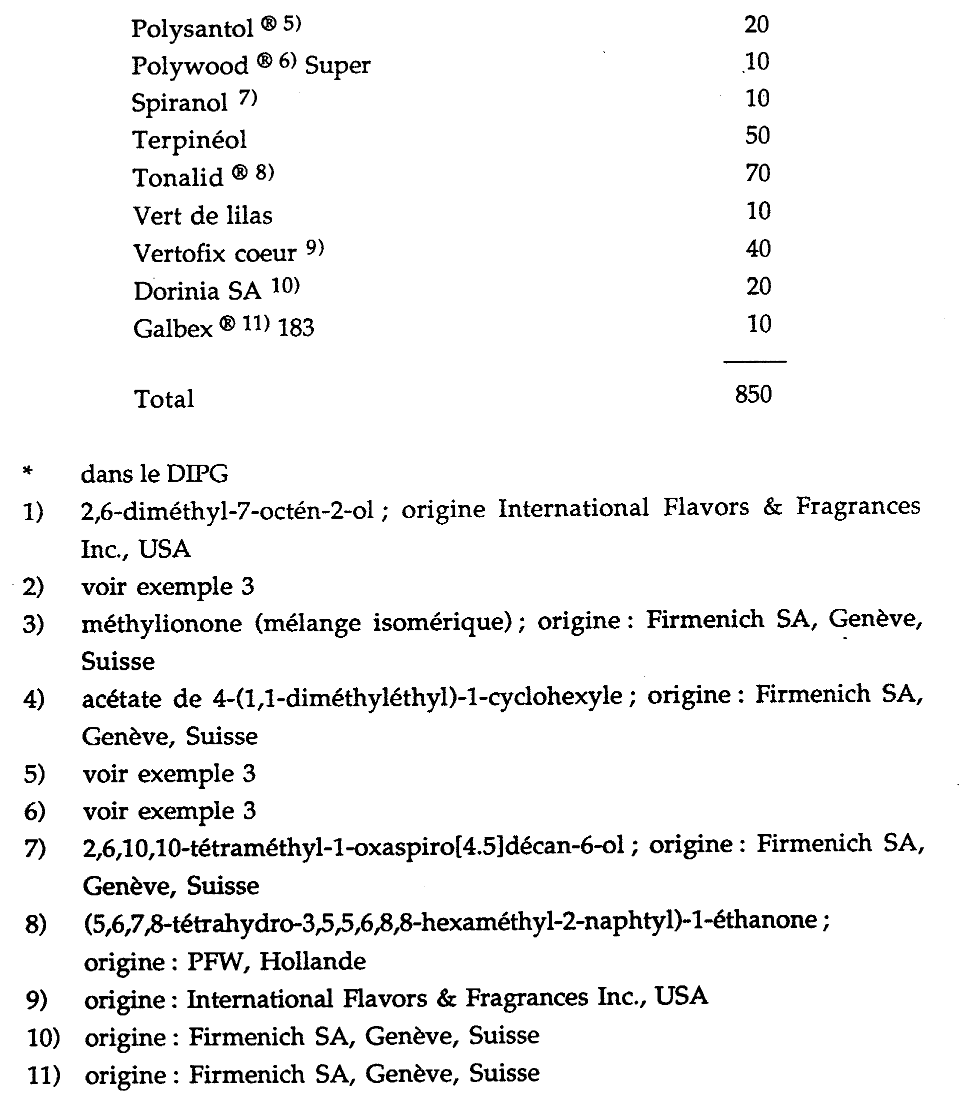

A cette composition de base de type floral ont été ajoutées 150 parties en poids de 3-(4-tert-butyl-2-méthyl-1-phényl)propanal pour préparer une nouvelle composition A, 100 parties en poids du même composé pour préparer une nouvelle composition B et 150 parties en poids de 3-(4-tert-butyl-1-phényl)-2-méthylpropanal pour préparer une composition C.
Ces trois compositions ont ensuite été utilisées à concentration identique pour préparer trois échantillons, respectivement A, B et C d'un détergent en poudre parfumé.
Un panel de 7 parfumeurs, évaluant ces trois échantillons de détergent à l'aveugle a montré une nette préférence pour les échantillons A et B dont l'odeur a été jugée plus fleurie-poudrée, plus puissante et plus élégante que celle de l'échantillon C.
Trois lots standards de textiles ont ensuite été lavés séparément dans trois machines avec les échantillons A, B et C et l'odeur des textiles a été évaluée à l'aveugle par un panel de 6 experts parfumeurs. L'évaluation a eu lieu sur des textiles humides, à la sortie de la machine, ainsi qu'après 24 h de séchage à l'air.
Les parfumeurs ont unanimement préféré l'odeur des textiles traités avec l'échantillon A, aussi bien à l'état humide qu'après séchage, suivis de ceux traités avec l'échantillon B. L'odeur de ces deux lots a été trouvée nettement supérieure, du point de vue puissance et qualité, à celle des textiles lavés avec l'échantillon C, en dépit du fait que la concentration du composé selon l'invention dans l'échantillon B était inférieure à celle du 3-(4-tert-butyl-1-phényl)-2-méthylpropanal dans l'échantillon C.
L'odeur des linges humides lavés avec les échantillons A et B a été jugée beaucoup plus fleurie que celle des textiles traités avec l'échantillon C, alors que les textiles secs exhalaient une odeur beaucoup plus puissante avec une note poudrée-muguet et légèrement mimosa que l'on ne retrouvait pas dans les- textiles parfumés avec l'échantillon C.

### Exemples 7 - 16

On a procédé au parfumage des articles mentionnés ci-après dans les concentrations indiquées, par adjonction de 3-(4-tert-butyl-2-méthyl-1-phényl)propanal aux bases appropriées non parfumées:

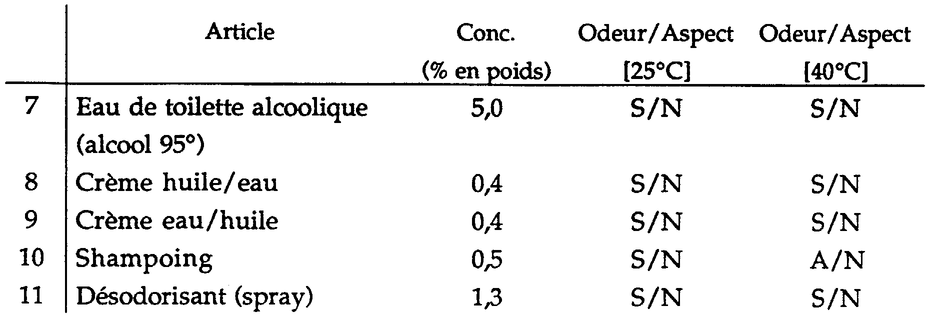

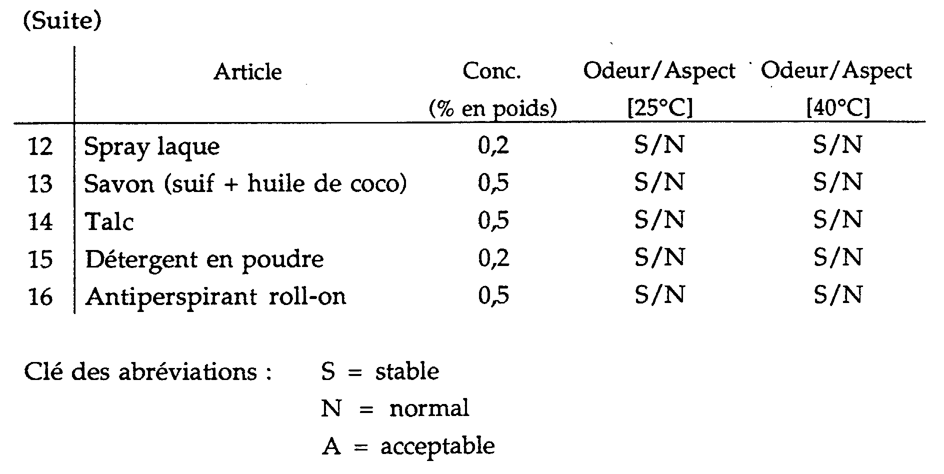

Les essais de parfumage et stabilité résumés dans le tableau ci-dessus ont montré que le 3-(4-tert-butyl-2-méthyl-1-phényl)propanal convient parfaitement au parfumage d'articles de consommation variés et peut trouver de ce fait un emploi étendu en parfumerie. Il a été observé qu'il couvrait efficacement l'odeur de la base, là où il y avait lieu, et qu'il impartissait à ces produits une odeur florale, muguet, poudrée, mimosa très agréable et élégante.

### Exemple 17

### Test de stabilité sur mouillette

On a effectué des tests de stabilité sur mouillette, en comparant la performance du 3-(4-tert-butyl-2-méthyl-1-phényl)propanal selon l'invention (mouillette A) avec celle de ses deux analogues connus, à savoir le 3-(4-tert-butyl-1-phényl)propanal (mouillette B) et le 3-(4-tert-butyl-1-phényl)-2-méthylpropanal ou LILIAL® (mouillette C).
Pour ce faire, un panel de 4 experts parfumeurs a trempé des mouillettes dans des flacons contenant les composés susmentionnés à l'état pur, de façon à obtenir une zone imprégnée d'environ 1 cm dans chaque cas. Ces mouillettes ont ensuite été évaluées à l'aveugle, et leurs odeurs comparées, dans le temps, cette opération étant répétée tous les jours jusqu'à ce que les parfumeurs ne décèlent plus d'odeur sur aucune des mouillettes.
De l'avis des parfumeurs, au départ, la mouillette A développait une odeur florale où la connotation de type muguet, mimosa était nettement dominante. Elle exhalait en plus une note douce, anisique, poudrée.
La mouillette B avait une odeur florale, beaucoup plus verte et aldéhydée que celle de la mouillette A, plus agressive aussi, et la mouillette C possédait une odeur florale du même type de celle de la mouillette A, mais moins anisique et dépourvue du caractère de type mimosa.
L'évolution de l'intensité de l'odeur des trois mouillettes dans le temps, telle que jugée par les parfumeurs sur une échelle de valeurs allant de 0 à 10, est indiquée dans le tableau suivant (moyenne des quatre):

| Mouillette | 3 jours | 7 j | 12 j | 15 j | 30 j | 44 j |
|---|---|---|---|---|---|---|
| A | 5 | 5 | 4 | 3 | 3 | 3 |
| B | 8 | 4 | 1 | - | - | - |
| C | - | - | - | - | - | - |

Ainsi, il a été constaté que l'odeur de la mouillette C diminuait fortement d'intensité dans les premières 24 h et n'était plus détectable à la fin de 3 jours. La mouillette B maintenait une odeur intense à la fin de 3 jours, qui s'estompait cependant rapidement dans la semaine qui suivait, alors que la mouillette A, dont l'intensité de l'odeur était au départ inférieure à celle de la mouillette B (de qualité odorante distincte de toute façon), conservait par la suite une intensité pratiquement stable et exhalait encore une fragrance parfaitement perceptible un mois et demi après avoir été trempée dans le composé selon l'invention. Par ailleurs, de l'avis des parfumeurs, la qualité de l'odeur de la mouillette A n'avait souffert d'aucune altération à la fin de cette période.

### Exemple 18

### Test de stabilité à l'oxydation par chromatographie en phase gazeuse (GC)

L'évolution qualitative décrite dans l'exemple précédent, sur la base de l'évaluation odorante des parfumeurs, a été parfaitement confirmée, de façon quantitative, à l'aide de mesures par chromatographie en phase gazeuse (GC). On a procédé comme suit.
On a déposé sur trois mouillettes standard (7x147 mm) une goutte de, respectivement, 3-(4-tert-butyl-2-méthyl-1-phényl)propanal (mouillette A), 3-(4-tert-butyl-1-phényl)propanal (mouillette B) et de 3-(4-tert-butyl-1-phényl)-2-méthylpropanal (mouillette C).
La zone des mouillettes ainsi imprégnée (- 20 mm) a été coupée et plongée pendant 1h dans du CH₂Cl₂ (1 ml) contenu dans des tubes à essai fermés, en agitant occasionnellement.
Avant d'injecter les solutions dans un appareil GC, on a ajouté de la bis-(triméthylsilyl)-acétamide (Aldrich, 4 gouttes, ∼30 mg) à chacune des trois solutions, pour former l'ester triméthylsilylique correspondant à l'acide formé par oxydation à l'air de l'aldéhyde extrait de chacune des mouillettes. n a, en effet, été constaté que le signal GC desdits esters était nettement moins large que celui de l'acide correspondant, et permettait donc une intégration beaucoup plus précise.
Les trois solutions ont ensuite été injectées dans un appareil GC à périodes régulières, adaptées à la vitesse d'oxydation observée pour chacun des trois aldéhydes susmentionnés. Les signaux correspondant à l'aldéhyde et à l'ester triméthylsilylique (proportionnel à la quantité d'acide formé) on été intégrés et les résultats obtenus représentés sur le graphique à la Figure 1.
Sur ce graphique, le pourcentage d'aldéhyde et d'acide correspondant sont représentés en fonction du temps. Les courbes représentées traduisent les valeurs moyennes obtenues pour deux expériences distinctes, effectuées avec chacun des composés dont les structures sont représentées.
Il résulte clairement de la Figure 1 que le composé selon l'invention, à savoir le 3-(4-tert-butyl-2-méthyl-1-phényl)propanal, est beaucoup plus stable à l'oxydation à l'air que son isomère connu, le 3-(4-tert-butyl-1-phényl)-2-méthylpropanal ou LILIAL®, lequel, au bout d'environ 4 jours, s'est transformé à 80% en l'acide correspondant, lequel est pratiquement inodore.
Lorsque l'on compare le composé de l'invention à son homologue inférieur connu, à savoir le 3-(4-tert-butyl-1-phényl)propanal ou BOURGEONAL® (origine : Naarden Intl, Hollande), on voit de nouveau clairement que ce dernier, bien que plus stable que le LILIAL®, à la fin d'une vingtaine de jours, s'est transformé à 70% en l'acide correspondant, alors que l'aldéhyde selon la présente invention reste à ∼90% stable.
Il convient de noter que ces résultats ne pouvaient être attribués à des différences de volatilité et/ou polarité des composés de l'invention par rapport à son isomère connu, le LILIAL®. Nous avons, en effet, mesuré les temps de rétention de ces deux composés sur deux types de colonnes de chromatographie gaz/liquide (GLC), ainsi que les valeurs R_{f} par chromatographie en couche mince (TLC). Les résultats présentés ci-après montrent qu'il n'y a pas de différences significatives dans ces valeurs.

| | Lilial® | Composé de l'invention |
|---|---|---|
| Temps rétention GLC colonne silice | 5,27 | 6,06 |
| [min] colonne Carbowax | 11,20 | 13,46 |
| TLC : R_{f} sur SiO₂ (éluant : CH₂Cl₂) | 0,60 | 0,56 |

## Revendications

1. Composé de formule 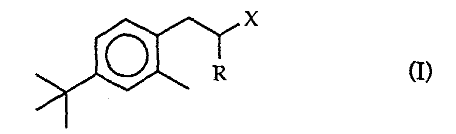 dans laquelle le symbole X représente un groupe - CHO ou un groupe acétal de formule 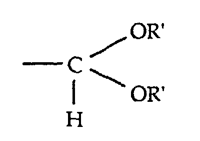 les symboles R', pris individuellement, représentant chacun un radical hydrocarbure de C₁ à C₄, linéaire ou ramifié, saturé ou insaturé, ou pris ensemble, représentant un radical alkylène de C₁ à C₄, éventuellement substitué, et le symbole R représente un atome d'hydrogène ou un radical méthyle.

2. 3-(4-tert-Butyl-2-méthyl-1-phényl)propanal.

3. Utilisation d'un composé selon la revendication 1 ou 2 à titre d'ingrédient parfumant.

4. Composition parfumante ou artide parfumé contenant en tant qu'ingrédient actif un composé selon la revendication 1 ou 2.

5. Article parfumé selon la revendication 4, sous forme d'un parfum ou d'une eau de toilette, d'un savon, d'un gel de douche ou bain, d'un shampoing ou d'un produit après-shampoing, d'une préparation cosmétique, d'un désodorisant corporel ou d'air ambiant, d'un détergent ou adoucissant textile ou d'un produit d'entretien.

6. Procédé de préparation d'un composé de formule (I) selon la revendication 1, caractérisé en ce qu'on fait réagir un alcool de formule 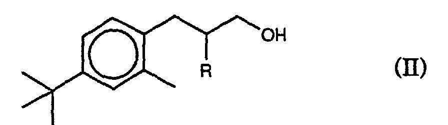 dans laquelle le symbole R représente un atome d'hydrogène ou un radical méthyle, avec un agent oxydant connu pour sa capacité d'oxyder la fonction alcool en aldéhyde, dans un solvant organique inerte, pour obtenir un aldéhyde de formule (I) et en ce que, le cas échéant, on acétalyse ce dernier, de façon connue en soi.

7. Procédé selon la revendication 6, caractérisé en ce que l'alcool de formule (II) est obtenu par hydrogénolyse, en présence d'un catalyseur, d'une oxépine de formule 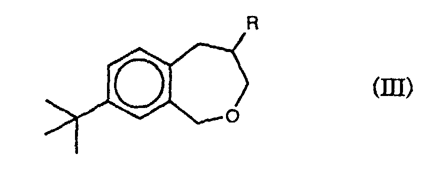 dans laquelle le symbole R représente un atome d'hydrogène ou un radical méthyle.

8. Composé de formule

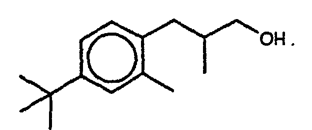

9. Composé de formule 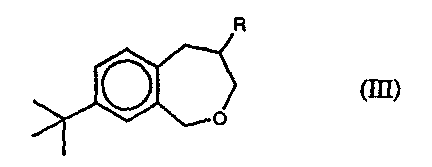 dans laquelle le symbole R représente un atome d'hydrogène ou un radical méthyle.

10. Procédé pour la préparation d'un composé de formule (I) selon la revendication 1, caractérisé en ce qu'on soumet un aldéhyde de formule 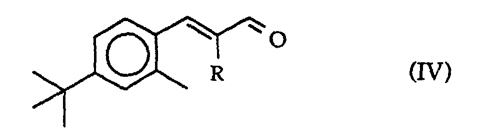 dans laquelle R a le sens indiqué à la formule (I), à une hydrogénation catalytique, dans un solvant organique inerte et, le cas échéant, on acétalyse, de façon connue en soi, l'aldéhyde (I) ainsi obtenu.

11. Procédé selon la revendication 10, caractérisé en ce que l'aldéhyde (IV) est le 3-(4-tert-butyl-1-phényl)-2-propénal, lequel est obtenu par réaction du 4-tert-butyl-1-(diméthoxyméthyl)-2-méthylènebenzène avec l'éthyl vinyl éther, en présence de chlorure de zinc et d'acide phosphorique.

12. Composé de formule 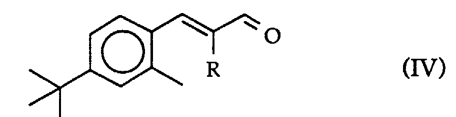 dans laquelle R représente un atome d'hydrogène ou un radical méthyle.

## Patentansprüche

1. Verbindung mit der Formel 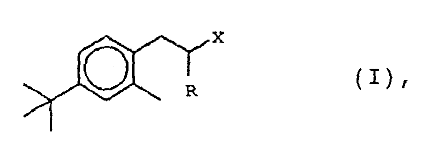 bei welcher das Symbol 1 X für eine -CHO-Gruppe oder eine Acetalgruppe mit der Formel 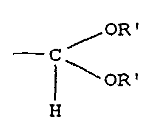 steht, wobei die Symbole R' jeweils für sich genommen für einen C₁-C₄-Kohlenwasserstoffrest stehen, der unverzweigt oder verzweigt, gesättigt oder ungesättigt ist, oder zusammen genommen für einen gegebenenfalls substituierten C₁-C₄-Alkylenrest, stehen,- und das Symbol R für ein Wasserstoffatom oder einen Methylrest steht.

2. 3-(4-*tert*-Butyl-2-methyl-1-phenyl)propanal.

3. Verwendung einer Verbindung nach Anspruch 1 oder 2 als Riechstoffbestandteil.

4. Duftzusammensetzung bzw. parfümierter Artikel, welche als aktiven Bestandteil eine Verbindung nach Anspruch 1 oder 2 enthalten.

5. Parfümiertes Produkt nach Anspruch 4 in Form von Parfum oder Eau de Toilette, Seife, Dusch- bzw. Badegel, Shampoo oder eines nach der Haarwäsche zu verwendenden Pflegemittels, eines kosmetischen Präparates, von Körper- oder Raumluftdeodorant, eines Reinigungsmittels oder Weichspülers für Textilien oder eines Universalhaushaltreinigers.

6. Verfahren zur Herstellung einer Verbindung mit der Formel (I) nach Anspruch 1, dadurch gekennzeichnet, daß man einen Alkohol mit der Formel 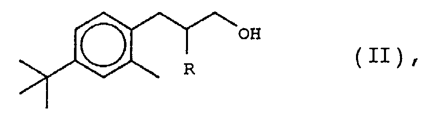 in welcher das Symbol R für ein Wasserstoffatom oder einen Methylrest steht, in einem inerten organischen Lösungsmittel mit einem Oxidationsmittel umsetzt, welches für seine Fähigkeit bekannt ist, die Alkoholgruppe zu einem Aldehyd zu oxidieren, um einen Aldehyd mit der Formel (I) herzustellen, und dieser Aldehyd gegebenenfalls auf an sich bekannte Weise acetalisiert wird.

7. Verfahren nach Anspruch 6, dadurch gekennzeichnet, daß der Alkohol mit der Formel (II) in Gegenwart eines Katalysators durch Hydrogenolyse eines Oxepins mit der Formel 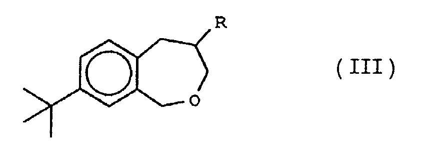 hergestellt wird, in welcher das Symbol R für ein Wasserstoffatom oder einen Methylrest steht.

8. Verbindung mit der Formel

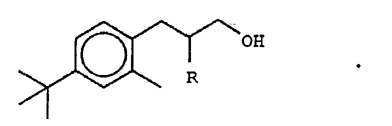

9. Verbindung mit der Formel 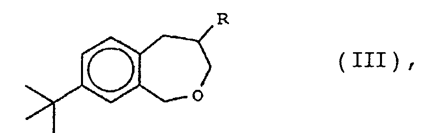 in welcher das Symbol R für ein Wasserstoffatom oder einen Methylrest steht.

10. Verfahren zur Herstellung einer Verbindung mit der Formel (I) nach Anspruch 1, dadurch gekennzeichnet, daß ein Aldehyd mit der Formel 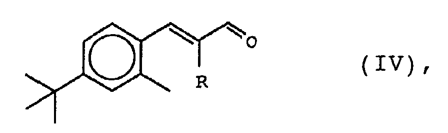 bei dem R die bezüglich Formel (I) angegebene Bedeutung hat, einer katalytischen Hydrierung in einem inerten organischen Lösungsmittel unterzogen und der derart hergestellte Aldehyd (I) gegebenenfalls auf an sich bekannte Weise acetalisiert wird.

11. Verfahren nach Anspruch 10, dadurch gekennzeichnet, daß der Aldehyd (IV) 3-(4-*tert*-Butyl-1-phenyl)-2-propenal ist, welcher durch Umsetzung von 4-tert-Butyl-1-(dimethoxymethyl)-2-methylenbenzol mit Ethylvinylether in Gegenwart von Zinkchlorid und Phosphorsäure hergestellt wird.

12. Verbindung mit der Formel 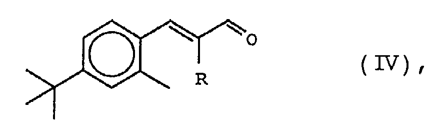 in welcher R für ein Wasserstoffatom oder einen Methylrest steht.

## Claims

1. Compound of formula 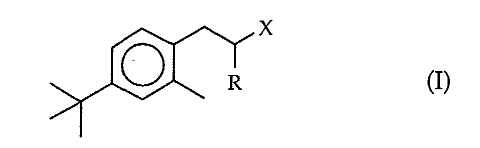 wherein symbol X represents a - CHO group or an acetal group of formula 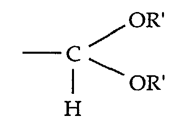 in which symbols R', taken separately, represent each a C₁ to C₄, linear or branched, saturated or unsaturated, hydrocarbon radical or, taken together, represent a C₁ to C₄ alkylene radical, optionally substituted, and symbol R represents a hydrogen atom or a methyl radical.

2. 3-(4-tert-Butyl-2-methyl-1-phenyl)propanal.

3. Use of a compound according to claim 1 or 2 as a perfuming ingredient.

4. Perfuming composition or perfumed article containing as active ingredient a compound according to claim 1 or 2.

5. Perfumed article according to claim 4, in the form of a perfume or a cologne, a soap, a bath or shower gel, a shampoo or an after-shampoo product, a cosmetic preparation, a body or air deodorant, a detergent or fabric softener, or a household product.

6. Process for the preparation of a compound of formula (I) according to claim 1, characterized in that an alcohol of formula 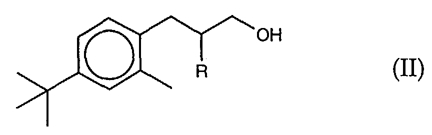 wherein symbol R represents a hydrogen atom or a methyl radical, is reacted with an oxidizing agent capable of oxidizing the alcohol function into aldehyde, in an inert organic solvent, to provide an aldehyde of formula (I) and in that, if necessary, the latter is then acetalyzed in a generally known manner.

7. Process according to claim 6, characterized in that the alcohol of formula (II) is obtained by hydrogenolysis, in the presence of a catalyst, of an oxepine of formula 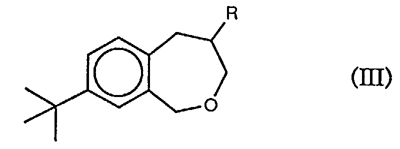 wherein symbol R represents a hydrogen atom or a methyl radical.

8. Compound of formula

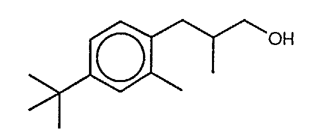

9. Compound of formula 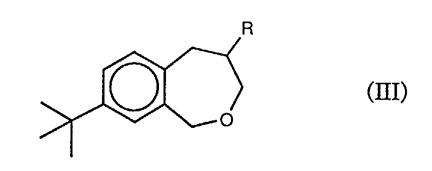 wherein symbol R represents a hydrogen atom or a methyl radical.

10. Process for the preparation of a compound of formula (I) according to claim 1, characterized in that an aldehyde of formula 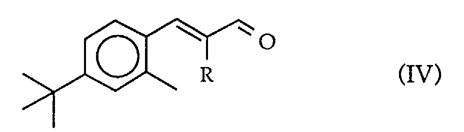 wherein R has the meaning indicated in formula (I), is subjected to catalytic hydrogenation in an inert organic solvent and, if necessary, the aldehyde thus obtained is acetalyzed in a generally known manner.

11. Process according to claim 10, characterized in that aldehyde (IV) is 3-(4-tert-butyl-1-phenyl)-2-propenal, which is obtained by reacting 4-tert-butyl-1-(dimethoxymethyl)-2-methylenebenzene with ethyl vinyl ether, in the presence of zinc chloride and phosphoric acid.

12. Compound of formula 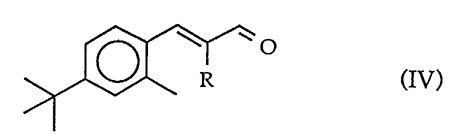 wherein R represents a hydrogen atom or a methyl radical.
